# EUROPEAN PATENT APPLICATION

(11) **EP 4 835 055 A1**
(43) Date of publication of application: **04.11.2026**
(21) Application number: 24911600.5
(22) Date of filing: 30.12.2024
(51) Int. Cl.: C12N 5/0783, A61K 35/17, A61P 37/00

(54) **METHOD FOR PREPARING INKT CELLS, AND USE**

(30) Priority: 29.12.2023 CN 202311864194
(71) Applicant: SPH Biotherapeutics (Shanghai) Limited, Shanghai 200072 (CN); SPH Biotherapeutics (HK) Limited, New Territories Hong Kong 999077 (HK)
(72) Inventor: QUAN, Dexian, Shanghai 200072 (CN); HUA, Jian, Shanghai 200072 (CN); ZHANG, Hua, Hong Kong 999077 (HK)
(74) Representative: Elzaburu S.L.P.
(86) International application number: PCT/CN2024/143622
(87) International publication number: WO 2025/140686

(57) **Abstract**

The present application relates to a method for preparing iNKT cells. The method comprises the steps of dividing human peripheral blood mononuclear cells (PBMCs) into two parts, then culturing one part by using a culture medium containing one or more iNKT cell agonists, and mixing cells of the two parts.

## Description

### Technical Field

The present application relates to the field of biomedicine and pharmaceuticals, and specifically to a method for preparing iNKT cells, a medium for culturing iNKT cells, and their applications.

### Background Art

Invariant natural killer T cells (iNKT cells) are innate-like CD1d-restricted T cells expressing an invariant T cell receptor (iTCR). They undergo a unique differentiation program in the thymus and acquire NK-like properties, including potent cytotoxicity, rapid cytokine secretion, and efficient trafficking into tissues. iNKT cells are among the earliest cell types to respond to pathogenic stimuli, and their cytokine production helps determine the course of the immune response. Due to their anti-tumor efficacy, iNKT cells have recently become a potential target for immunotherapy.

However, iNKT cells are present in very low numbers in the human body-approximately 1% of circulating T cells in human peripheral blood; up to about 10-25% in human adipose tissue; and very rarely in the human liver. Therefore, there is an urgent need to develop a method for preparing iNKT cells to rapidly obtain large quantities of iNKT cells for the treatment of related diseases.

### Summary of the Invention

The present application provides a method for preparing iNKT cells, which can significantly increase the expansion fold of iNKT cells-reaching approximately 80,000-fold after about 24 days of culture, with cell viability above 80%.

In one aspect, the present application provides a method for preparing iNKT cells, comprising the following steps:
(1) Obtaining human peripheral blood mononuclear cells (PBMCs);
(2) Dividing the PBMCs into two portions, wherein the first portion comprises Res cells and the second portion comprises Sti cells;
(3) Culturing the Sti cells in a medium containing one or more iNKT cell agonists; and
(4) Adding the Res cells to the Sti cells for co-culture.

In certain embodiments, the method is performed under serum-free and exogenous trophoblast cell-free conditions.

In certain embodiments, the iNKT cell agonist used in the method is selected from α-GalCer or 7DW8-5.

In certain embodiments, the concentration of the iNKT cell agonist in the method is 100 ng/ml to 5000 ng/ml.

In certain embodiments, the iNKT cell agonist used in the method is α-GalCer.

In certain embodiments, the concentration of α-GalCer in the method is 100 ng/ml to 5000 ng/ml.

In certain embodiments, the culture duration in step (3) is 0.5-3 h.

In certain embodiments, the culture duration in step (4) is 10-40 days.

In certain embodiments, step (4) further comprises culturing the mixed Res cells and Sti cells in a medium containing one or more factors selected from the group consisting of IL-2, IL-4, IL-7, IL-15, IL-21, TNF-α, IFN-γ, and IGF.

In certain embodiments, step (4) further comprises culturing the mixed Res cells and Sti cells in a medium containing IGF, IL-7, and IL-15.

In certain embodiments, step (4) further comprises culturing the mixed Res cells and Sti cells in a medium containing IL-7 and IL-15.

In certain embodiments, step (4) further comprises culturing the mixed Res cells and Sti cells in a medium containing IL-15.

In certain embodiments, the concentration of the factors in the method is 5 ng/ml to 100 ng/ml.

In certain embodiments, the concentration of the factor IGF in the method is 10 ng/ml to 1000 ng/ml.

In certain embodiments, the concentration of the factor IL-7 in the method is 5 ng/ml to 100 ng/ml.

In certain embodiments, the concentration of the factor IL-15 in the method is 5 ng/ml to 100 ng/ml.

In certain embodiments, the medium in the method further comprises Serum replacement.

In certain embodiments, the medium in the method comprises a basal medium selected from one or more of the following: TexMACS medium, X-VIVO media, CTS OpTmizer T Cell Expansion SFM, CTS AIM V SFM, SCGM medium, OptiVitro T Cell Serum-free Medium, OptiVitro UniEx T Cell Serum-free Medium, ImmunoCult^{™}-XF T Cell Expansion Medium, and PRIME-XV medium.

In certain embodiments, the basal medium in the method is TexMACS medium.

In certain embodiments, the ratio of Res cells to Sti cells in the method is 1:0.01 to 1:100.

In certain embodiments, the method is carried out under culture conditions of 37 °C ± 1 °C.

In certain embodiments, the method is carried out under culture conditions of 5 ± 0.5% CO₂.

In another aspect, the present application provides a medium that is a single-component medium comprising components (1), (2), and (3):
wherein the components are:
Component (1): basal medium;
Component (2): one or more iNKT cell agonists; and
Component (3): one or more factors selected from the group consisting of IL-2, IL-4, IL-7, IL-15, IL-21, TNF-α, IFN-γ, and IGF.

In another aspect, the present application provides a medium that is a combination of multiple stepwise media, wherein the stepwise media comprise the following media and/or combinations thereof:
(a): Components (1) and (2),
(b): Components (1) and (3), and
(c): Components (1), (2), and (3);
wherein the components are:
Component (1): basal medium;
Component (2): one or more iNKT cell agonists; and
Component (3): one or more factors selected from the group consisting of IL-2, IL-4, IL-7, IL-15, IL-21, TNF-α, IFN-γ, and IGF.

In certain embodiments, the basal medium in the medium is selected from one or more of the following: TexMACS medium, X-VIVO media, CTS OpTmizer T Cell Expansion SFM, CTS AIM V SFM, SCGM medium, OptiVitro T Cell Serum-free Medium, OptiVitro UniEx T Cell Serum-free Medium, ImmunoCult^{™}-XF T Cell Expansion Medium, and PRIME-XV medium.

In certain embodiments, the basal medium in the medium is TexMACS medium.

In certain embodiments, the iNKT cell agonist in the medium is selected from α-GalCer or 7DW8-5.

In certain embodiments, the iNKT cell agonist in the medium is α-GalCer.

In certain embodiments, the concentration of α-GalCer in the medium is 100 ng/ml to 5000 ng/ml.

In certain embodiments, the concentration of the factors in the medium is 5 ng/ml to 100 ng/ml.

In certain embodiments, the factors in the medium are IGF, IL-7, and IL-15.

In certain embodiments, the factors in the medium are IL-7 and IL-15.

In certain embodiments, the factor in the medium is IL-15.

In certain embodiments, the concentration of IGF in the medium is 10 ng/ml to 1000 ng/ml.

In certain embodiments, the concentration of IL-7 in the medium is 5 ng/ml to 100 ng/ml.

In certain embodiments, the concentration of IL-15 in the medium is 5 ng/ml to 100 ng/ml.

In certain embodiments, the medium may further comprise Serum replacement.

In certain embodiments, the medium does not contain serum.

In another aspect, the present application provides a method for expansion culture of iNKT cells, comprising using the method described herein and/or using the medium described herein.

In another aspect, the present application provides a composition comprising human peripheral blood mononuclear cells and the medium described herein.

In another aspect, the present application provides a composition comprising iNKT cells and the medium described herein.

In another aspect, the present application provides a composition comprising human peripheral blood mononuclear cells and iNKT cells, and the medium described herein.

In another aspect, the present application provides the use of the composition described herein in the manufacture of a medicament for the prevention, alleviation, and/or treatment of a disease and/or disorder.

Other aspects and advantages of the present application will be readily apparent to those skilled in the art from the detailed description below. Only exemplary embodiments of the present application are shown and described in the detailed description below. As will be recognized by those skilled in the art, the disclosure herein enables modifications to the specific embodiments disclosed without departing from the spirit and scope of the invention described herein. Accordingly, the descriptions in the drawings and specification of the present application are merely exemplary and not restrictive.

### Brief Description of the Drawings

The specific features of the invention described herein are set forth in the appended claims. The characteristics and advantages of the invention described herein can be better understood by reference to the exemplary embodiments and drawings detailed below. A brief description of the drawings is as follows:
Figure 1 shows the proportion of initial iNKT cells in PBMCs and the proportion of iNKT cells after culture, as described herein.
Figure 2A shows the expansion fold of iNKT cells at different culture times using the method described herein versus a control method.
Figure 2B shows the viability of iNKT cells at different culture times using the method described herein versus a control method.
Figure 2C shows the proportion of iNKT cells at different culture times using the method described herein versus a control method.
Figure 3A shows the proportion of iNKT cells after culture with cytokines IL-7 and IL-15, as described herein.
Figure 3B shows the proportion of iNKT cells after culture with cytokines IL-7, IL-15, and IGF, as described herein.
Figure 4 shows the cytotoxicity evaluation of iNKT cells described herein.
Figures 5A and 5B show the proportions of CD4 and CD8 subpopulations in iNKT cells cultured with different factors, as described herein.
Figure 5C shows the cytotoxicity evaluation of iNKT cells cultured with different factors, as described herein.

### Detailed Description of Specific Embodiments

The following specific examples illustrate the embodiments of the invention described herein. Those skilled in the art can readily understand other advantages and effects of the invention from the disclosure provided in this specification.

### Definition of Terms

As used herein, the term "iNKT cells" generally refers to a specialized subset of T cells involved in innate immunity, characterized by an invariant TCRα receptor. iNKT cells can recognize antigens in the presence of CD1d molecules and specifically recognize α-GalCer; they can be labeled using CD1d/α-GalCer tetramers. iNKT cells can differentiate into NKT1, NKT2, and NKT17 subsets, analogous to Th1, Th2, and Th17 subsets. iNKT cells contain large amounts of cytotoxic cytokines (such as IL-4, IL-10, IFN-γ, etc.), far exceeding those in NK cells and T cells; upon stimulation, iNKT cells can rapidly release cytokines to kill tumor cells.

As used herein, the term "cell agonist" generally refers to a natural or synthetic molecule that binds to a receptor on a cell and elicits a response. Natural cell agonists can be hormones or neurotransmitters. Synthetic cell agonists can be drugs that mimic natural agonists. As used herein, "iNKT cell agonists" can bind to receptors on cells. For example, the receptor can be a receptor on iNKT cells, such as CD1d. Using iNKT cell agonists can promote the proliferation/differentiation/activation of iNKT cells. For example, iNKT cell agonists can be α-GalCer or 7DW8-5.

In the present application, the term "human peripheral blood mononuclear cells" or "PBMC" generally refers to mononuclear cells in peripheral blood. For example, any blood cells with a round nucleus (i.e., lymphocytes, monocytes, or macrophages). These blood cells are key components of the immune system in resisting infections and adapting to invaders. The lymphocyte population consists of CD4+ and CD8+ T cells, B cells, natural killer cells, CD14+ monocytes, and basophils/neutrophils/eosinophils/dendritic cells. Typically, these cells are isolated from whole blood using FICOLL^{TM} (a hydrophilic polysaccharide that stratifies blood), wherein monocytes and lymphocytes form a buffy coat layer beneath the plasma layer.

In the present application, the term "diagnosis" generally refers to detecting a disease or disorder, or determining the status or extent of a disease or disorder, and also includes detecting causative agents of a disease or disorder, assessing the therapeutic efficacy of drug treatment, or predicting response patterns to drug therapy or xenobiotics. The diagnostic methods described herein may be used independently or in combination with other diagnostic and/or staging methods for specific diseases or disorders known in the medical field.

In the present application, the term "prevention" generally refers to methods indicating prevention, inhibition, or reduction of the likelihood of occurrence or recurrence of a disease or condition (e.g., cancer). It also refers to delaying the onset or recurrence of a disease or condition, or delaying the appearance or recurrence of symptoms of a disease or condition. "Prevention" and similar terms also include reducing the intensity, impact, symptoms, and/or burden of said disease or condition prior to its onset or recurrence.

In the present application, the term "treatment" generally refers to any beneficial or desirable effect on symptoms or lesions of a disease or pathological condition, and may even include minimal reduction of one or more measurable markers of the treated disease or condition (e.g., cancer). Treatment may optionally involve reduction or improvement of symptoms of a disease or condition, or delay in progression of the disease or condition. "Treatment" does not necessarily indicate complete eradication or cure of a disease or condition or its associated symptoms.

In the present application, the term "proliferation" generally refers to the process by which starting cells generate multiple cellular individuals through division. The multiple cellular individuals may be cells of the same type or different types. The starting cells for proliferation need not be identical to the cells produced by proliferation. For example, proliferating cells may arise from the growth and differentiation of an initial cell population.

In the present application, the term "differentiation" generally refers to the process by which non-specific or less-specific cells acquire characteristics of specific cell types. Differentiated or differentiation-inducible cells are those occupying a more specific position within a cell lineage.

In the present application, the term "composition" generally refers to a product comprising specified amounts of specified ingredients, as well as any product directly or indirectly resulting from the combination of specified amounts of specified ingredients. In the present application, said composition may further comprise other inactive ingredients, such as carriers, excipients, adjuvants, stabilizers, etc.

In the present application, the term "ex vivo" generally refers to procedures involving manipulation of cells, tissues, and/or organs that have been removed from a living organism. In certain embodiments, said cells, tissues, and/or organs may be reintroduced into the original organism or transferred into another organism by certain methods.

In the present application, the term "in vitro" generally refers to removing or releasing a part of an organism from the organism itself.

In the present application, the term "comprising" generally carries the meaning of including, encompassing, containing, or incorporating. In certain contexts, it also conveys the meaning of "being" or "consisting of."

In the present application, the term "and/or" should be understood to mean any one, two, multiple, or any combination thereof among the alternatives.

In the present application, the term "about" generally refers to variation within a range of 0.5%-10% above or below a specified value, for example, within ±0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the specified value.

### Detailed Description of the Invention

### Methods for preparing iNKT cells

In one aspect, the present application provides a method for preparing iNKT cells, said method comprising the following steps:
(1) Obtaining human peripheral blood mononuclear cells (PBMC);
(2) Dividing PBMC into two portions, wherein the first portion comprises Res cells and the second portion comprises Sti cells;
(3) Culturing Sti cells in a medium containing one or more iNKT cell agonists; and
(4) Adding Res cells to Sti cells for co-culture.

In the present application, said method may involve culturing PBMC in contact with iNKT cell agonists to promote preparation/expansion/differentiation/culture of iNKT cells.

In the present application, said iNKT cell agonists may bind to receptors on cells and elicit a response.

For example, they may promote preparation/expansion/differentiation/culture of iNKT cells.

In the present application, said iNKT cell agonists may be natural or synthetic. Natural agonists may be hormones or neurotransmitters, while synthetic agonists may be drugs structurally similar to natural agonists.

For example, iNKT cell agonists may be full agonists, also known as direct-binding agonist drugs, which bind directly to the receptor at the same binding site as the natural ligand. For example, iNKT cell agonists may be partial agonists, also known as indirect-binding agonist drugs, which enhance binding of the natural ligand to the receptor site. These drugs produce a delayed response; in certain cases, partial agonists may act as antagonists by competing with full agonists for the same receptor.

For example, iNKT cell agonists may bind to CD1d. For example, iNKT cell agonists may be α-GalCer. For example, iNKT cell agonists may be 7DW8-5.

In the present application, the concentration of iNKT cell agonists may be 100 ng/mL-5000 ng/mL.

For example, it may be 100 ng/mL, about 150 ng/mL, about 200 ng/mL, about 250 ng/mL, about 300 ng/mL, about 350 ng/mL, about 400 ng/mL, about 450 ng/mL, about 500 ng/mL, about 550 ng/mL, about 600 ng/mL, about 650 ng/mL, about 700 ng/mL, about 750 ng/mL, about 800 ng/mL, about 850 ng/mL, about 900 ng/mL, about 950 ng/mL, or about 1000 ng/mL.

In the present application, the duration for culturing Sti cells with iNKT cell agonists may be 0.5-3 h.

For example, it may be about 0.5 h, about 0.55 h, about 0.6 h, about 0.65 h, about 0.7 h, about 0.75 h, about 0.8 h, about 0.85 h, about 0.9 h, about 0.95 h, about 1 h, about 1.1 h, about 1.15 h, about 1.2 h, about 1.25 h, about 1.3 h, about 1.35 h, about 1.4 h, about 1.45 h, about 1.5 h, about 1.55 h, about 1.6 h, about 1.65 h, about 1.7 h, about 1.75 h, about 1.8 h, about 1.85 h, about 1.9 h, about 1.95 h, about 2 h, about 2.1 h, about 2.15 h, about 2.2 h, about 2.25 h, about 2.3 h, about 2.35 h, about 2.4 h, about 2.45 h, about 2.5 h, about 2.55 h, about 2.6 h, about 2.65 h, about 2.7 h, about 2.75 h, about 2.8 h, about 2.85 h, about 2.9 h, about 2.95 h, or about 3 h.

In the present application, the duration for adding Res cells to Sti cells for culture may be 10-40 days.

For example, it may be about 5-10 days, about 7-10 days, about 7-15 days, about 7-20 days, about 10-21 days, about 10-30 days, about 15-20 days, about 20-25 days, about 20-30 days, about 25-30 days, or about 30-40 days.

In the present application, when adding Res cells to Sti cells for culture, one or more substances may be added to the culture system, including but not limited to: nutrients/extracts, growth factors, hormones, cytokines, and/or medium additives.

For example, one or more of the following substances may be added, including but not limited to serum replacement, glutamine, NEAA (non-essential amino acids), ascorbic acid, epidermal growth factor (EGF), acidic fibroblast growth factor (aFGF), basic fibroblast growth factor (bFGF), leukemia inhibitory factor (LIF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transforming growth factor β (TGF-β), bone morphogenetic protein (BMP4), vascular endothelial growth factor (VEGF), transferrin, insulin, selenium, various interleukins (e.g., IL-1 to IL-18), various colony-stimulating factors (e.g., granulocyte/macrophage colony-stimulating factor (GM-CSF)), various interferons (e.g., IFN-γ), stem cell factor (SCF), thrombopoietin (TPO), erythropoietin (EPO), N2 supplement, B27 supplement, and Fms-related tyrosine kinase 3 ligand (Flt3). Said added substances may be sourced from commercial suppliers or may be natural or recombinant.

For example, IL-2, IL-4, IL-7, IL-15, IL-21, TNF-α, IFN-γ, and IGF may be added. For example, IGF, IL-7, and IL-15 may be added. For example, Serum replacement may be further added.

In the present application, the concentration of one or more added substances may be 5 ng/mL-100 ng/mL.

For example, it may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IL-2 may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IL-4 may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IL-7 may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IL-15 may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IL-21 may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said TNF-α may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IFN-γ may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

For example, the concentration of said IGF may be about 5 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, 55 ng/mL, about 60 ng/mL, about 65 ng/mL, about 70 ng/mL, about 75 ng/mL, about 80 ng/mL, about 85 ng/mL, about 90 ng/mL, about 95 ng/mL, or about 100 ng/mL.

In the present application, a basal medium may be added when culturing cells using said method. Said basal medium may comprise any medium known in the art.

For example, said basal medium may comprise one or more of the following: TexMACS medium, X-VIVO media, CTS OpTmizer T Cell Expansion SFM, CTS AIM V SFM, SCGM medium, OptiVitro T Cell Serum-free Medium, OptiVitro UniEx T Cell Serum-free Medium, ImmunoCult^{™}-XF T Cell Expansion Medium, PRIME-XV medium. For example, said basal medium may be TexMACS medium.

In the present application, said Res cells and Sti cells may be mixed at an appropriate ratio to achieve preparation of iNKT cells.

For example, the ratio of Res cells to Sti cells may be about 1:0.01 to 1:100. For example, it may be about 1:0.5, about 1:1.25, about 1:1.5, about 1:1.75, about 1:2, about 1:2.25, about 1:2.5, about 1:2.75, about 1:3, about 1:5, about 1:10, about 1:15, or about 1:30.

In the present application, said method is performed under culture conditions of 37°C ± 1°C.

For example, about 34.5°C, about 35°C, about 35.5°C, about 36°C, about 36.5°C, about 37°C, about 37.5°C, about 38°C, about 38.5°C, about 39°C, or about 39.5°C.

In the present application, said method may be performed under culture conditions of 5% ± 0.5% CO₂.

For example, about 3% CO₂, about 3.5% CO₂, about 4% CO₂, about 4.5% CO₂, about 5% CO₂, about 5.5% CO₂, about 6% CO₂, about 6.5% CO₂, about 7% CO₂, or about 7.5% CO₂.

In the present application, said method may be performed under serum-free conditions.

In the present application, said method may be performed under serum-containing conditions.

In the present application, said method may be performed in the absence of exogenous trophoblast cells.

In the present application, said method may be performed with or without exogenous trophoblast cells.

### Culture Media

In another aspect, the present application provides a single-component culture medium for cell culture, comprising components (1), (2), and (3); wherein said components are:
Component (1): basal medium;
Component (2): one or more iNKT cell agonists; and
Component (3): one or more factors selected from the group consisting of: IL-2, IL-4, IL-7, IL-15, IL-21, TNF-α, IFN-γ, and IGF.

In another aspect, the present application provides a culture medium comprising a combination of multiple stepwise media, said stepwise media comprising the following media and/or combinations thereof:
(a): Components (1) and (2),
(b): Components (1) and (3), and
(c): Components (1), (2), and (3);
wherein said components are:
Component (1): basal medium;
Component (2): one or more iNKT cell agonists; and
Component (3): one or more factors selected from the group consisting of: IL-2, IL-4, IL-7, IL-15, IL-21, TNF-α, IFN-γ, and IGF.

For example, said factors may be IGF, IL-7, and IL-15. For example, said factors may be IL-7 and IL-15. For example, said factor may be IL-15. For example, Serum replacement may be further added to the medium.

In the present application, the medium may be used alone or in combination with other media, and the proportions of the respective media can be arbitrarily adjusted during use.

For example, the single-component medium may be used together with other media. For example, multiple stepwise media may be used individually, one or more of the multiple stepwise media may be used in combination, or one or more of the multiple stepwise media may be combined with other media for use.

In the present application, the type and concentration of the iNKT cell agonists, the one or more factors, and the basal medium in the medium may be adaptively adjusted as described in the iNKT cell preparation method of the present application to achieve the purpose of preparing/expanding/differentiating/culturing iNKT cells.

In the present application, one or more substances may also be added to the medium, including but not limited to: nutrients/extracts, growth factors, hormones, cytokines, and/or medium additives.

For example, one or more of the following substances may be added, including but not limited to serum replacement, glutamine, NEAA (non-essential amino acids), ascorbic acid, epidermal growth factor (EGF), acidic fibroblast growth factor (aFGF), basic fibroblast growth factor (bFGF), leukemia inhibitory factor (LIF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transforming growth factor β (TGF-β), bone morphogenetic protein (BMP4), vascular endothelial growth factor (VEGF), transferrin, insulin, selenium, various interleukins (e.g., IL-1 to IL-18), various colony-stimulating factors (e.g., granulocyte/macrophage colony-stimulating factor (GM-CSF)), various interferons (e.g., IFN-γ), stem cell factor (SCF), thrombopoietin (TPO), erythropoietin (EPO), N2 supplement, B27 supplement, and Fms-related tyrosine kinase 3 ligand (Flt3). The source of the added substances is not limited; they may be commercially available or natural or recombinant.

In the present application, serum may be further added to the medium.

In the present application, the types and sources of cells that can be cultured in the medium are not limited.

For example, they may be of natural origin or modified. For example, they may be of mammalian origin or non-mammalian origin. For example, they may be a single component or a mixture of multiple cell types. For example, the cells may be PBMCs, iNKT cells, or a mixture of PBMCs and iNKT cells.

### Composition

In another aspect, the present application provides a composition comprising PBMCs and the medium described herein.

In another aspect, the present application provides a composition comprising iNKT cells and the medium described herein.

In another aspect, the present application provides a composition comprising PBMCs, iNKT cells, and the medium described herein.

In the present application, the composition may be used for preparing/expanding/differentiating/culturing iNKT cells.

### Use

In another aspect, the present application provides the use of the composition in the manufacture of a medicament for the prevention, alleviation, and/or treatment of diseases and/or disorders.

In another aspect, the present application also provides a culture platform for obtaining iNKT cells, which comprises the method and the medium described herein.

In another aspect, the present application also provides a method for preventing and/or treating a disease, comprising administering iNKT cells to a subject in need thereof, wherein the iNKT cells are obtained using the method, the medium, the composition, or the culture platform described herein.

For example, the administered iNKT cells may be pharmaceutically formulated by any conventional method. For example, the active ingredient may be mixed or diluted with a carrier, excipient, or diluent. Examples of suitable carriers, excipients, or diluents include lactose, dextrose, sucrose, sorbitol, mannitol, glycine, polyethylene glycol, starch, gum arabic, alginic acid, gelatin, calcium phosphate, calcium silicate, cellulose, methylcellulose, microcrystalline cellulose, polyvinylpyrrolidone, water, methylparaben, propylparaben, talc, magnesium stearate, and mineral oil. The formulation may additionally contain, for example, fillers, anti-caking agents, lubricants, wetting agents, flavoring agents, emulsifiers, preservatives, etc. The composition of the present invention may be formulated by any known method in the art to provide rapid, sustained, or delayed release of the active ingredient upon administration to a patient.

In the present application, administration of the cells may be performed via injection (e.g., intramuscular, intravenous, intraperitoneal, subcutaneous) or by other methods such as infusion to ensure entry into the bloodstream in an effective form. The cells may also be administered via intratumoral, peritumoral, intralesional, or perilesional routes to exert local and systemic therapeutic effects. For example, administration may be performed via local or intravenous injection.

In the present application, the administered dose of the cells may be a single dose or multiple doses. For example, the actual administered amount of the cells may be determined based on various relevant factors, such as the type of disease; route of administration; age, sex, and/or weight of the patient; and severity of the patient's symptoms.

Without being bound by any theory, the examples below are provided solely to illustrate the preparation methods and uses of the present application and are not intended to limit the scope of the invention disclosed herein.

### Experimental Materials

| Name | Supplier | Catalog Number |
|---|---|---|
| FICOLL PAQUE PREMIUM | Cytiva | 175-44-202 |
| TexMACS medium | Miltenyi | 170-076-309 |
| α-GalCer | KRN7000 | Funakoshi |
| Recombinant Human Interleukin-7 | PrimeGene | 101-07 |
| Recombinant Human Interleukin-15 | PrimeGene | 101-15 |
| Recombinant Human Insulin-like Growth Factor-1 | PrimeGene | 105-01 |
| CTS Immune Cell Serum Replacement | Thermo | A2596101 |

### Example 1 Preparation of iNKT Cells from Human Peripheral Blood Mononuclear Cells (PBMCs)

### 1.1 Isolation of Peripheral Blood Mononuclear Cells (PBMCs)

For cryopreserved PBMC samples, cells were thawed in a 37°C water bath and held for subsequent use.

For fresh blood samples, PBMCs were isolated from blood samples using Ficoll density gradient centrifugation as follows:
1) Measure the volume of the blood sample and dilute it with 2-4 volumes of isotonic buffer (PBS/saline);
2) Prepare centrifuge tubes and add an equal volume of Ficoll to each tube as the diluted blood sample;
3) Gently layer an equal volume of the diluted leukapheresis product onto the Ficoll in each tube and perform density gradient centrifugation at 400 × g for 30 min at room temperature with acceleration set to 1 and deceleration set to 1;
4) After centrifugation, collect the buffy coat cells into centrifuge tubes, add buffer, and pellet the cells by centrifugation at 500 × g for 10 min at room temperature with acceleration set to 9 and deceleration set to 9;
5) Resuspend the cell pellet in buffer and wash by centrifugation at 500 × g for 10 min at room temperature, then hold for subsequent use.

### 1.2 Counting and Analysis of PBMCs

1) Count PBMCs using the trypan blue exclusion method;
2) Determine the proportion of iNKT cells in PBMCs by flow cytometry.

### 1.3 Preparation of Res Cells

1) Based on the PBMC count, transfer two-thirds of the total cell number into centrifuge tubes and remove the supernatant by centrifugation at 350 × g for 10 min;
2) Resuspend the cell pellet in TexMACS medium and transfer the cells into appropriate culture flasks for incubation at 37°C in a 5% CO₂ environment until needed.

### 1.4 Preparation of Sti Cells

1) Prepare TexMACS medium containing α-GalCer;
2) Based on the PBMC count, transfer one-third of the total cell number into centrifuge tubes and remove the supernatant by centrifugation at 350 × g for 10 min;
3) Resuspend the cell pellet in the above TexMACS medium containing α-GalCer and transfer the cells into appropriate culture flasks for incubation at 37°C in a 5% CO₂ environment for 0.5-3 h;
4) After incubation, collect the cells into centrifuge tubes and pellet the cells by centrifugation at 350 × g for 10 min;
5) Resuspend the cell pellet in TexMACS medium and wash the cells twice by centrifugation at 350 × g for 10 min;
6) Resuspend the cell pellet in TexMACS medium and hold for subsequent use.

### 1.5 Co-culture of Res Cells and Sti Cells

1) Mix the prepared Res cells and Sti cells and transfer them into an appropriate culture vessel for static culture;
2) Add IGF to the culture system to a final concentration of approximately 100 ng/mL; add IL-7 and IL-15 to final concentrations of approximately 5 ng/mL each;

On days 3 and 5 of culture, add serum replacement at 5% of the total culture volume.

### 1.6 Cell Pelleting and Medium Change

1) Prepare complete TexMACS medium containing 5% serum replacement, 10 ng/mL IL-7, and 10 ng/mL IL-15;
2) On day 7 of culture, collect all cell suspension into centrifuge tubes and pellet the cells by centrifugation at 350 × g for 10 min;
3) Resuspend the cell pellet in the above complete medium and culture at 37°C in a 5% CO₂ environment.

### 1.7 Cell Passaging

1) Starting from day 7, sample and count cells every 2-3 days and determine the proportion of iNKT cells by flow cytometry;
2) Starting from day 7, every 2-3 days adjust the cell density to 1E6/mL-2E6/mL using complete TexMACS medium containing 5% serum replacement, 10 ng/mL IL-7, and 10 ng/mL IL-15 for passaging.

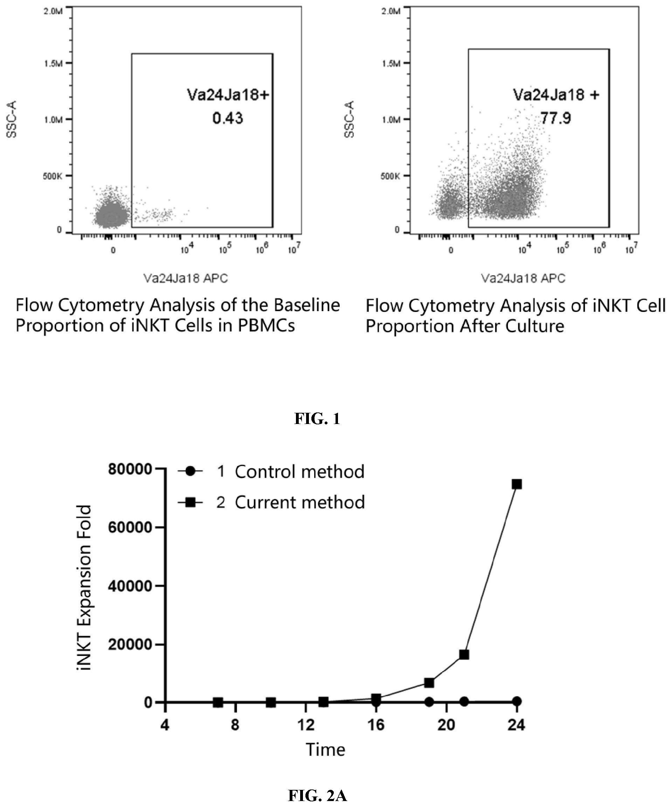

The experimental results are shown in Figure 1:
The initial proportion of iNKT cells in PBMCs was 0.43%; after 13 days of culture using the method of Example 1, the proportion of iNKT cells reached 77.9%.

These results indicate that the method described in the present application can promote the preparation/expansion/differentiation/culture of iNKT cells.

### Example 2 Evaluation of Different iNKT Cell Culture Methods

### 2.1 Evaluation of iNKT Cell Preparation Using Different Culture Methods (I)

Current Method: The method described in Example 1
Control Method:
   1) Directly initiate culture with PBMCs (without splitting PBMCs into two parts; excluding steps 1.3, 1.4, and 1.5 of Example 1) in medium containing α-GalCer, IL-7, and IL-15;
   2) All other culture steps are identical to those in Example 1.

The experimental results are shown in Figures 2A-2C:
In Figure 2A, using the current method (the method of Example 1), the expansion fold of iNKT cells increased rapidly around days 16-24 of culture, reaching approximately 80,000-fold by day 24 of culture. In contrast, with the control method (the method of Example 2.1), the expansion fold showed essentially no change by day 24 of culture.

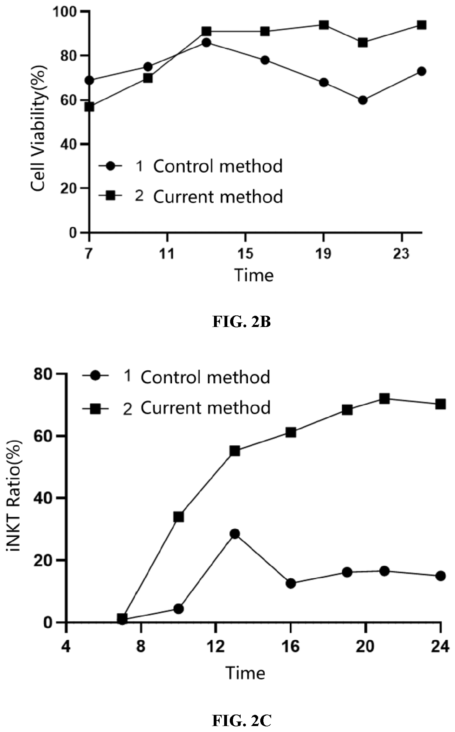

In Figure 2B, using the current method (the method of Example 1), the viability of iNKT cells remained above 80% around day 12 of culture, significantly higher than that achieved with the control method (the method of Example 2.1); moreover, by approximately day 23 of culture, the viability of iNKT cells was still maintained above 80%.

In Figure 2C, using the current method (the method of Example 1), the proportion of iNKT cells continuously increased, exceeding 50% after day 12 of culture and even reaching 70% during days 16-24 of culture, which is significantly higher than the 20% obtained with the control method (the method of Example 2.1).

The above results indicate that the method described in the present application can promote the preparation/expansion/differentiation/culture of iNKT cells. Around day 24 of culture, the method described in the present application can significantly enhance the expansion fold, viability, and cellular proportion of iNKT cells, enabling the rapid acquisition of a large number of iNKT cells within a short period.

### 2.2 Evaluation of iNKT cell production efficacy using different culture methods (II)

Under otherwise identical culture conditions, different cytokines were added to the culture system in step 1.5 of Example 1, and the remaining steps were performed as described in the other steps of Example 1. The proportion of iNKT cells was measured on day 10 of culture.

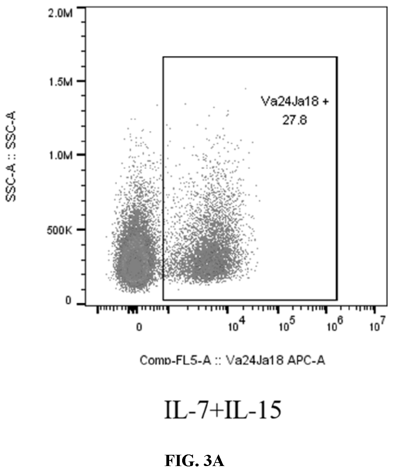

The experimental results are shown in Figures 3A-3B:
In Figure 3A, the cytokines added were IL-7 and IL-15, resulting in an iNKT cell proportion of 27.8%.

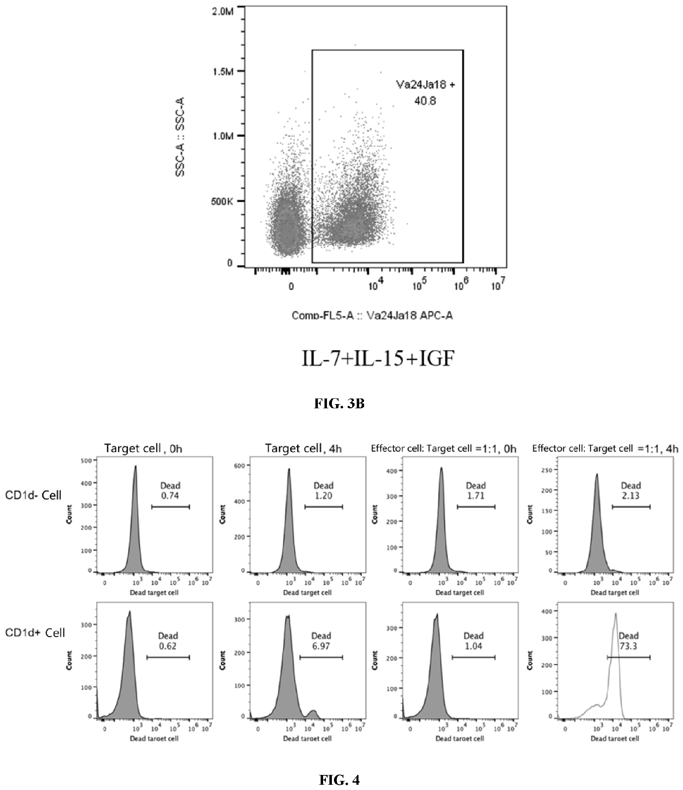

In Figure 3B, the cytokines added were IL-7, IL-15, and IGF, resulting in an iNKT cell proportion of 40.8%.

The above results indicate that the method described in Example 1 of the present application can promote the preparation/expansion/differentiation/culture of iNKT cells.

### Example 3 Verification of iNKT Cell Cytotoxic Function

Target cells and iNKT cells were prepared, and the target cells were fluorescently labeled using Cell Trace dye. The labeled target cells and iNKT cells were counted and mixed at a ratio of 1:1, then cultured in a 37°C, 5% CO₂ incubator. After 4 hours of co-culture, all cells were collected, centrifuged, washed, and stained with Annexin V. Following staining, flow cytometry was used to determine the percentage of Annexin V-positive cells within the Cell Trace fluorescence-positive cell population, which represents the killing rate of iNKT cells against target cells.

The experimental results are shown in Figure 4:
iNKT cells cultured using the method described in the present application effectively killed target cells; after 4 hours of co-culture with target cells, approximately 73.3% of the target cells were killed.

The above results indicate that iNKT cells prepared by the method described in the present application exhibit good functional activity and can be used for treatment.

### Example 4 Evaluation of CD4 and CD8 Subsets and Cytotoxic Efficacy of iNKT Cells Cultured with Different Cytokines

1) Under otherwise identical culture conditions, different cytokines were added to the culture system in steps 1.5, 1.6, and 1.7 of Example 1, and the proportions of CD4+, CD8+, CD4-CD8-, and CD4+CD8+ iNKT cell subsets were measured at different time points during culture.

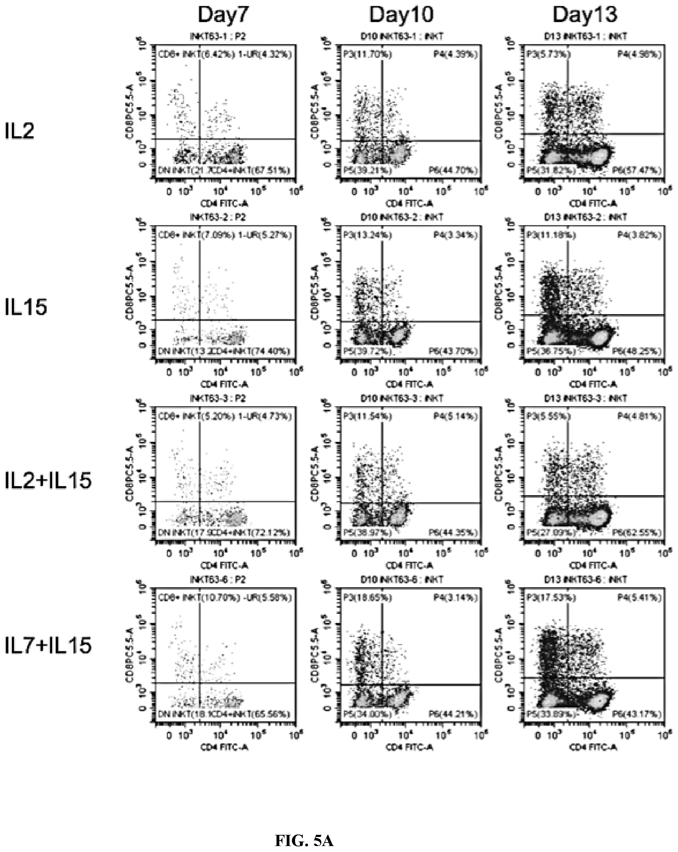

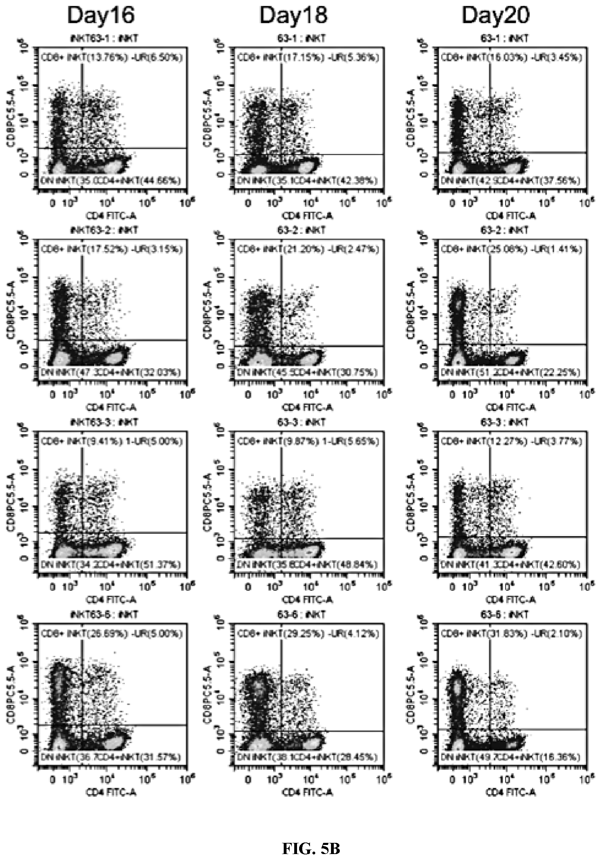

The experimental results are shown in Figures 5A and 5B:
On day 20, cultures supplemented with IL-15 or the IL-7 + IL-15 cytokine combination exhibited a CD4+ subset proportion of approximately 20%, with the corresponding non-CD4+ subsets (CD8+, CD4-CD8-, and CD4+CD8+ cell subsets) accounting for approximately 80%. In contrast, cultures supplemented with IL-2 or the IL-2 + IL-15 cytokine combination showed a CD4+ subset proportion of approximately 40%, with the corresponding non-CD4+ subsets accounting for approximately 60%.

2) Using the iNKT cell cytotoxicity assay described in Example 3, iNKT cells cultured with different cytokines were co-cultured with target cells at an effector-to-target ratio of 1:5 for 4 hours, after which the killing rate of target cells was determined.

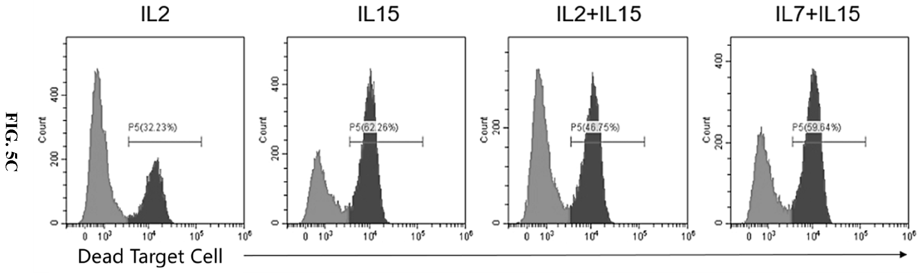

The experimental results are shown in Figure 5C:
iNKT cells cultured with the IL-15 or IL-7 + IL-15 cytokine combinations exhibited target cell killing rates of 62.2% and 59.6%, respectively, which are higher than the killing rates of 32.2% and 46.7% observed for iNKT cells cultured with the IL-2 or IL-2 + IL-15 cytokine combinations.

## Claims

1. A method for preparing iNKT cells, comprising the following steps:
(1) obtaining human peripheral blood mononuclear cells (PBMCs);
(2) dividing the PBMCs into two portions, wherein the first portion comprises Res cells and the second portion comprises Sti cells;
(3) culturing the Sti cells in a medium containing one or more iNKT cell agonists; and
(4) adding the Res cells to the Sti cells for culture.

2. The method of claim 1, wherein the method is performed under serum-free and exogenous trophoblast cell-free conditions.

3. The method of any one of claims 1-2, wherein the iNKT cell agonist is selected from α-GalCer or 7DW8-5.

4. The method of any one of claims 1-3, wherein the concentration of the iNKT cell agonist is 100 ng/ml-5000 ng/ml.

5. The method of any one of claims 1-4, wherein the iNKT cell agonist is α-GalCer.

6. The method of any one of claims 1-5, wherein the concentration of α-GalCer is 100 ng/ml-5000 ng/ml.

7. The method of any one of claims 1-6, wherein the culture duration of step (3) is 0.5-3 h.

8. The method of any one of claims 1-7, wherein the culture duration of step (4) is 10-40 days.

9. The method of any one of claims 1-8, wherein step (4) further comprises culturing the mixed Res cells and Sti cells in a medium containing one or more factors selected from the group consisting of: IL-2, IL-4, IL-7, IL-15, IL-21, TNF-α, IFN-γ, and IGF.

10. The method of any one of claims 1-9, wherein step (4) further comprises culturing the mixed Res cells and Sti cells in a medium containing IGF, IL-7, and IL-15.

11. The method of any one of claims 1-9, wherein step (4) further comprises culturing the mixed Res cells and Sti cells in a medium containing IL-7 and IL-15.

12. The method of any one of claims 1-9, wherein step (4) further comprises culturing the mixed Res cells and Sti cells in a medium containing IL-15.

13. The method of any one of claims 9-12, wherein the concentration of the factors are 5 ng/ml-100 ng/ml.

14. The method of any one of claims 9-10, wherein the concentration of the factor IGF is 10 ng/ml-1000 ng/ml.

15. The method of any one of claims 9-11, wherein the concentration of the factor IL-7 is 5 ng/ml-100 ng/ml.

16. The method of any one of claims 9-12, wherein the concentration of the factor IL-15 is 5 ng/ml-100 ng/ml.

17. The method of any one of claims 9-16, wherein the medium further comprises Serum replacement.

18. The method of any one of claims 9-17, wherein the medium comprises a basal medium selected from one or more of the following: TexMACS medium, X-VIVO media, CTS OpTmizer T Cell Expansion SFM, CTS AIM V SFM, SCGM medium, OptiVitro T Cell Serum-free Medium, OptiVitro UniEx T Cell Serum-free Medium, ImmunoCult^{™}-XF T Cell Expansion Medium, and PRIME-XV medium.

19. The method of claim 18, wherein the basal medium is TexMACS medium.

20. The method of any one of claims 1-19, wherein the ratio of Res cells to Sti cells is 1:0.01-1:100.

21. The method of any one of claims 1-20, wherein the method is performed under culture conditions of 37°C ± 1°C.

22. The method of any one of claims 1-21, wherein the method is performed under culture conditions of 5% ± 0.5% CO₂.

23. A medium, wherein the medium is a single-component medium comprising components (1), (2), and (3); wherein said components are:
Component (1): basal medium;
Component (2): one or more iNKT cell agonists; and
Component (3): one or more factors selected from the group consisting of: IL-2, IL-4, IL-7, IL-15, IL-21, TNF-α, IFN-γ, and IGF.

24. A medium, wherein the medium is a combination of multiple stepwise media, said stepwise media comprising the following media and/or combinations thereof:
(a): components (1) and (2),
(b): components (1) and (3), and
(c): components (1), (2), and (3);
wherein said components are:
Component (1): basal medium;
Component (2): one or more iNKT cell agonists; and
Component (3): one or more factors selected from the group consisting of: IL-2, IL-4, IL-7, IL-15, IL-21, TNF-α, IFN-γ, and IGF.

25. The medium of any one of claims 23-24, wherein the basal medium is selected from one or more of the following: TexMACS medium, X-VIVO media, CTS OpTmizer T Cell Expansion SFM, CTS AIM V SFM, SCGM medium, OptiVitro T Cell Serum-free Medium, OptiVitro UniEx T Cell Serum-free Medium, ImmunoCult^{™}-XF T Cell Expansion Medium, and PRIME-XV medium.

26. The medium of any one of claims 23-25, wherein the basal medium is TexMACS medium.

27. The medium of any one of claims 23-26, wherein the iNKT cell agonist is selected from α-GalCer or 7DW8-5.

28. The medium of any one of claims 23-27, wherein the iNKT cell agonist is α-GalCer.

29. The medium of any one of claims 27-28, wherein the concentration of α-GalCer is 100 ng/ml-5000 ng/ml.

30. The medium of any one of claims 23-29, wherein the concentration of the factors are 5 ng/ml-100 ng/ml.

31. The medium of any one of claims 23-30, wherein the factors are IGF, IL-7, and IL-15.

32. The medium of any one of claims 23-31, wherein the factors are IL-7 and IL-15.

33. The medium of any one of claims 23-32, wherein the factor is IL-15.

34. The medium of any one of claims 23-33, wherein the concentration of IGF is 10 ng/ml-1000 ng/ml.

35. The medium of any one of claims 23-34, wherein the concentration of IL-7 is 5 ng/ml-100 ng/ml.

36. The medium of any one of claims 23-35, wherein the concentration of IL-15 is 5 ng/ml-100 ng/ml.

37. The medium of any one of claims 23-36, wherein the medium may further comprise Serum replacement.

38. The medium of any one of claims 23-37, wherein the medium does not contain serum.

39. The medium of any one of claims 23-38, wherein the medium is used for preparing iNKT cells.

40. A method for expansion culture of iNKT cells, comprising the method of any one of claims 1-22 and/or using the medium of any one of claims 23-39.

41. A composition comprising human peripheral blood mononuclear cells and the medium of any one of claims 23-39.

42. A composition comprising iNKT cells and the medium of any one of claims 23-39.

43. A composition comprising human peripheral blood mononuclear cells and iNKT cells, and the medium of any one of claims 23-39.

44. Use of the composition of any one of claims 41-43 in the manufacture of a medicament for prevention, alleviation, and/or treatment of disease and/or disorder.
